# EUROPEAN PATENT SPECIFICATION

(11) **EP 1 820 533 B1**
(45) Date of publication and mention of the grant of the patent: **02.11.2011**
(21) Application number: 05814574.9
(22) Date of filing: 06.12.2005
(51) Int. Cl.: A61N 1/30, A61N 1/32

(54) **ION-TOPHORETIC APPARATUS**
IONTOPHORETISCHES GERÄT
APPAREIL IONTOPHORÉTIQUE

(30) Priority: 09.12.2004 JP 2004357313; 30.06.2005 US 172372
(43) Date of publication of application: 22.08.2007
(73) Proprietor: TTI ellebeau, Inc., Tokyo 150-0022 (JP)
(72) Inventor: TANIOKA, Akihiko, 1450061 (JP); MATSUMURA, Akihiko, Tokyo 140-0002 (JP); MATSUMURA, Takehiko, Tokyo 140-0002 (JP); NAKAYAMA, Mizuo, Tokyo 140-0002 (JP); AKIYAMA, Hidero, Tokyo 140-0002 (JP)
(74) Representative: Schäfer, Matthias W.
(86) International application number: PCT/JP2005/022395
(87) International publication number: WO 2006/062108

(56) References cited:
- EP-A- 0 571 712
- EP-A- 0 774 272
- EP-A- 0 900 576
- EP-A- 0 904 779
- EP-A- 1 043 043
- EP-A- 1 050 319
- EP-A- 1 059 097
- EP-A- 1 433 495
- EP-A- 1 440 707
- WO-A1-03/037425
- JP-A- 09 201 420
- JP-A- 2004 188 188
- US-A- 5 169 383
- US-A- 5 628 729
- US-A1- 2003 135 150

## Description

### FIELD OF THE INVENTION

The present invention relates to an iontophoresis device for administering dissociated drug ions through an ion-exchange membrane that selects ions of the same conductivity as that of the drug ions, capable of further enhancing the administration efficiency of the drug ions.

### BACKGROUND OF THE INVENTION

An iontophoresis device generally includes a working electrode structure holding drug ions dissociated to plus or minus ions and a nonworking electrode structure that functions as a counterpart of the working electrode structure. The drug ions are administered to a living body by the application of a voltage with the same polarity as that of the drug ions to the working electrode structure under the condition that both the structures are in contact with the skin of the living body (human being or animal).

In this case, the charge supplied to the working electrode structure is consumed by the movement of the drug ions to the living body and the release of biological counter ions (the ions which are present in the living body and charged in a conductivity type opposite to that of the drug ions) to the working electrocde structure, and the biological counter ions (e.g., Na⁺ and Cl⁻) having a small molecular weight and hence having a large mobility are released mainly from the living body. Thus, the ratio of the charge consumed by the release of biological counter ions increases, which makes it impossible to administer drug ions effectively.

Patent Documents 1 to 10 below disclose iontophoresis devices that have solved the above-mentioned problem.

More specifically, in each of the iontophoresis devices in Patent Documents 1 to 10, a working electrode structure is composed of an electrode, a drug holding part placed on a front side (in contact with the skin) of the electrode, and an ion-exchange membrane that is placed on a front side of the drug holding part and selectively passes ions with the same polarity as that of the drug ions held by the drug holding part, and the drug ions are administered through the ion-exchange membrane, whereby the release of biological counter ions is suppressed to enhance the administration efficiency of the drug.

In each of the iontophoresis devices in Patent Documents 1 to 10, the working electrode structure further includes an electrolyte solution holding part for holding an electrolyte solution in contact with the electrode, and an ion-exchange membrane that selectively passes ions having a conductivity type opposite to that of drug ions, thereby achieving the additional effects of preventing the drug ions from being decomposed by isolating the drug ions from the electrode and preventing the movement of H⁺ or OH⁻ ions generated at the electrode to the drug holding part and the skin interface of a living body.

Here, in the iontophoresis devices of Patent Documents 1 to 10, in order to facilitate the passage of drug ions with a relatively large molecular weight and effectively suppressing the release of biological counter ions from a living body, an ion-exchange membrane is used in which a porous film made of polyolefin, vinyl chloride-based resin, fluorine-based resin, or the like is filled with ion-exchange resin (resin provided with an ion-exchange function). As such a porous film has low affinity for the skin of a living body, it is difficult to keep the contact between the ion-exchange membrane and the skin of the living body in a satisfactory state during the administration of drug ions, and depending upon the site which a working electrode structure and a nonworking electrode structure are brought into contact with, the behavior of the living body (patient) during the administration, and the like, the administration efficiency of drug ions cannot be maintained at a sufficient level.

Therefore, the following inconvenience is caused. During the administration of the drug ions, it is necessary to interpose an electrolyte solution or the like between the ion-exchange membrane and the skin of the living body, or further keep pressing the working electrode structure and the nonworking electrode structure against the skin of the living body with some bias means.

The same kind of problem also arises in other devices that conduct the passage of a current to a living body, such as a low-frequency therapeutic equipment. In these devices, an adhesive film in which acrylic hydrogel is impregnated with an electrolyte solution is used, whereby the adhesion between the electrodes and the living body, and the conductivity are ensured.

However, when an adhesive film containing an electrolyte is interposed between the ion-exchange membrane and the skin, the function of the ion-exchange membrane of suppressing the release of biological counter ions from the skin degrades. Consequently, in the iontophoresis device for administering drug ions through an ion-exchange membrane, which is a target of the present invention, the administration efficiency of a drug cannot be enhanced by the use of this type of adhesive film.
Patent Document 1: JP 3030517 B
Patent Document 1: JP 2000-229128 A
Patent Document 1: JP 2000-229129 A
Patent Document 1: JP 2000-237326 A
Patent Document 1: JP 2000-237327 A
Patent Document 1: JP 2000-237328 A
Patent Document 1: JP 2000-237329 A
Patent Document 1: JP 2000-288097 A
Patent Document 1: JP 2000-288098 A
Patent Document 1: WO 03/037425

### DISCLOSURE OF INVENTION

### PROBLEMS TO BE SOLVED

The present invention has been achieved in view of the above-mentioned problems, and its object is to further enhance the administration efficiency of a drug in an iontophoresis device for administering drug ions to a living body through an ion-exchange membrane.

Another object of the present invention is to provide an iontophoresis device capable of enhancing the administration efficiency of drug ions without any inconvenience of interposing an electrolyte solution between the ion-exchange membrane and the skin of a living body or keeping pressing a working electrode structure and/or a nonworking electrode structure to the living body with some bias means.

### MEANS FOR SOLVING PROBLEMS

The present invention relates to an iontophoresis device for administering drug ions via a first ion-exchange membrane by applying a first conductivity voltage to a working electrode structure, said working electrode structure having a first ion-exchange membrane including a first porous film with a number of pores communicating a front surface and a back surface of the first porous film and an ion-exchange resin being filled in the pores of the first porous film, said first ion-exchange membrane selecting ions of a first conductivity; and a drug holding part placed on a back surface side of the first ion-exchange membrane and holding a drug solution containing drug ions charged in the first conductivity, and the above mentioned problem is solved by providing a first layer of a water-soluble polymer compound chemically bonded to a material constituting the first porous film on the front surface of the first porous film.

Although the mechanism for the remarkable enhancement of the administration efficiency of a drug according to the present invention has not been necessarily clarified, it is considered that the following may contribute to the mechanism. A water-soluble polymer compound chemically bonded to the front surface of the first porous film improves the affinity of an ion-exchange membrane with respect to the skin of a living body to some degree. Further, a layer of water bleeding out (oozing out) from the drug holding part is held at the first layer of the water-soluble polymer compound to enhance the conductivity between the ion-exchange resin and the skin. In addition, the first layer of the water-soluble polymer compound chemically bonded to the front surface of the porous film has a thickness from about one molecule to at most tens of molecules of the water-soluble polymer compound. Therefore, this layer does not substantially prevent the contact between the ion-exchange membrane and the skin, and in the same way as in the case where the layer of the water-soluble polymer compound is not present, the original function of the iontorophoresis device for administering drug ions through an ion-exchange membrane, i.e., the function of blocking the flow of biological counter ions from the skin while allowing the transfer of drug ions from the ion-exchange membrane to the skin is maintained.

The iontophoresis device of the present invention may further include, as a counterpart structure of the above-mentioned working electrode structure (structure supplied with a voltage of a conductivity opposite to that of the working electrode structure or grounded), a nonworking electrode structure that includes
a second ion-exchange membrane including a second porous film with a number of pores communicating a front surface and a back surface of the second porous film and an ion-exchange resin being filled in the pores of the second porous film, said second ion-exchange membrane selecting ions of a second conductivity opposite to the first conductivity; and
a first electrolyte solution holding part for holding an electrolyte solution, placed on a back surface side of the second ion-exchange membrane, and
a first layer of a water-soluble polymer compound chemically bonded to a material constituting the second porous film may be formed on the front surface of the second porous film.
With this feature, the effect of enhancing the adhesion with respect to the skin and the conductivity in the nonworking electrode structure is achieved, and the administration efficiency of a drug is enhanced further.

The water-soluble polymer compound in the present invention is a polymer compound with molecular weight of about 50 to 1,000 which dissolves easily in water. Examples of particularly preferably used water-soluble polymer compound include polyvinyl alcohol (PVA), carboxymethylcellulose, polyacrylic acid, poly-N-isopropylacrylamide, polyacrylamide, poly-N-methylacrylamide, polyvinylpyrrolidone, polymethacrylic acid, polyethylene glycol, polyethyleneimine, poly-N-dimethylaminoethyl methacrylate, and polypeptide.

As the porous film, thermoplastic resin such as polyethylene resin or polypropylene resin is used preferably. The chemical bond (covalent bond) between the porous film and each of the above-mentioned water-soluble polymer compounds can be formed by irradiating the porous film immersed in an aqueous solution in which the water-soluble polymer compound is dissolved, with a UV-ray, an electron beam, a γ-ray, or plasma.

Each of the water-soluble polymer compounds as illustrated above has a straight-chain structure, and by appropriately controlling the reaction condition for chemically bonding the water-soluble polymer compound to the front surface of the porous film, the water-soluble polymer compound can be bonded to the front surface of the porous film in a brush shape. This can remarkably enhance the administration efficiency of a drug in particular. Similarly, by appropriately controlling the reaction condition, the water-soluble polymer compound can be bonded to the front surface of the porous film in a layer shape. Even in this case, the administration efficiency of a drug can be enhanced to a degree comparable to that in the case where the water-soluble polymer compound is bonded in a brush shape.

The water-soluble polymer compound can be chemically bonded to the front surface of the porous film before/after the porous film is filled with the ion-exchange resin or before/after the ion-exchange group is introduced into the ion-exchange resin. It is preferable to chemically bond the water-soluble polymer compound to the front surface of the porous film after the porous film is filled with the ion-exchange resin, because it is preferable to prevent the adhesion of the water-soluble polymer compound to the walls of the pores of the porous film.

It is not necessarily required that the first layer of the water-soluble polymer compound are formed only on the front surface of the porous film and it is possible to form the first layer of the water-soluble polymer compound on both the front and back surfaces of the porous film.

Furthermore, according to the present invention, a second layer of a water-soluble polymer compound may be formed on the first layer of the water-soluble polymer compound chemically bonded to the front surface of the porous film.

The second layer absorbs water bled out from the drug holding part (or water supplied from outside if necessary) to be fluidized during the administration of drug ions. Therefore, when the working electrode structure and/or the nonworking electrode structure are brought into contact with the skin, the water-soluble polymer compound between the convex portion of the skin and the ion-exchange membrane moves to the space formed between the concave portion of the skin and the ion-exchange membrane, whereby the adhesion between the working electrode structure and/or the nonworking electrode structure, and the skin is further enhanced.

In order to make obtain sufficient fluidity during the use, it is preferable that the second layer is formed in such a way that the second layer does not chemically bond to the porous film, the ion-exchange resin, or the water-soluble polymer compound. For example, the second layer is preferably formed by coating such as spin coating or bar coating, or immersing the porous film in an aqueous solution of the water-soluble polymer compound.

The water-soluble polymer compound constituting the second layer includes polyvinyl alcohol (PVA), carboxymethylcellulose, polyacrylic acid, poly-N-isopropylacrylamide, polyacrylamide, poly-N-methylacrylamide, polyvinylpyrrolidone, polymethacrylic acid, polyethylene glycol, polyethyleneimine, poly-N-dimethylaminoethyl methacrylate, and polypeptide. The water-soluble polymer compound with molecular weight of about 1,000 to 1,000,000 which dissolves easily in water can be used without any particular limit.

### EFFECT OF THE INVENTION

According to the present invention, the adhesion and conductivity between a working electrode structure and/or a nonworking electrode structure, and the skin of a living body are enhanced, while the release of biological counter ions from the skin is minimized, whereby the administration efficiency of drug ions with respect to a living body can be enhanced.

Furthermore, as a result of the achievement of the above-mentioned functional effect, an iontophoresis device capable of administering drug ions stably to a living body with a high administration efficiency can be realized, without interposing an electrolyte solution between the working electrode structure and/or the nonworking electrode structure, and the skin of a living body, or without using bias means for keeping pressing the working electrode structure and/or the nonworking electrode structure against the living body, depending upon a site of the living body which the working electrode structure and/or the nonworking electrode structure is brought into contact with.

### BEST MODE FOR CARRYING OUT THE INVENTION

Hereinafter, an embodiment of the present invention will be described with reference to the drawings.

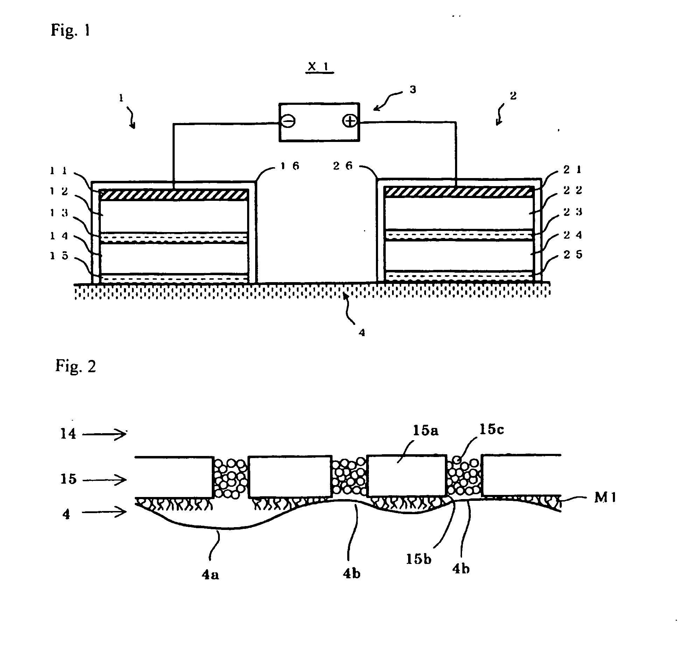

FIG. 1 is a schematic cross-sectional view showing a basic configuration of an iontophoresis device X1 according to the present invention.

In the following, for convenience of description, an iontophoresis device for administering a drug whose drug component is dissociated to minus ions (for example, ascorbic acid that is a vitamin agent, lipid A used as an adjuvant for vaccine) will be exemplified. In the case of an iontophoresis device for administering a drug whose drug component is dissociated to plus ions (for example, lidocaine that is an anesthetic agent, calnitine chloride that is a gastrointestinal disease therapeutic agent, pancuronium bromide that is a skeletal muscle relaxant, morphine hydrochloride that is an anesthetic agent), the polarity (plus or minus) of a power source, each electrode, and each ion-exchange membrane in the following description is reversed.

As shown, an iontophoresis device X1 of the present invention includes a working electrode structure 1, a nonworking electrode structure 2, and a power source 3, as main components (members). Reference numeral 4 denotes a skin (or a membrane).

The working electrode structure 1 includes an electrode 11 connected to a negative pole of the power source 3, an electrolyte solution holding part 12 for holding an electrolyte solution in contact with the electrode 11, an cation exchange membrane 13 placed on a front surface side of the electrolyte solution holding part 12, a drug holding part 14 placed on a front surface side of the cation exchange membrane 13, and an anion exchange membrane 15 placed on a front surface side of the drug holding part 14. The entire working electrode structure 1 is housed in a cover or a container 16 composed of a material such as a resin film or a plastic.

On the other hand, the nonworking electrode structure 2 includes an electrode 21 connected to a positive pole of the power source 3, an electrolyte solution holding part 22 for holding an electrolyte solution in contact with the electrode 21, a anion exchange membrane 23 placed on a front surface side of the electrolyte solution holding part 22, an electrolyte solution holding part 24 placed on a front surface side of the anion exchange membrane 23, and an cation exchange membrane 25 placed on a front surface side of the electrolyte solution holding part 24. The entire nonworking electrode structure 2 is housed in a cover or a container 26 composed of a material such as a resin film or a plastic.

In the iontophoresis device X1, those which are made of any conductive material can be used as the electrodes 11 and 21 without any particular limit. In particular, a inactive electrode composed of carbon, platinum, or the like is used preferably, and a carbon electrode without any possibility of the elution of metal ions and the transfer thereof to a living body can be used more preferably.

However, an active electrode such as a silver/silver chloride couple electrode in which the electrode 11 is made of silver chloride and the electrode 21 is made of silver can also be adopted.

For example, in the case of using the silver/silver chloride couple electrode, in the electrode 21 that is an anode (positive) pole, a silver electrode and chlorine ions (Cl⁻) easily react with each other to generate insoluble AgCl as represented by Ag⁺Cl⁻→AgCl+e⁻, and in the electrode 11 that is a cathode (negative pole), chlorine ions (Cl⁻) are eluted from a silver chloride electrode. Consequently, the following effects can be obtained: the electrolysis of water is suppressed, and the rapid acidification based on H⁺ ions at the anode, and the rapid basification based on OH⁻ ions at the cathode can be prevented.

In contrast, in the working electrode structure 1 and the nonworking electrode structure 2 in the iontophoresis device X1 in FIG. 1, owing to the function of the anion exchange membrane and/or the cation exchange membrane, the rapid basification based on OH⁻ ions in the electrolyte solution holding part 12 and the rapid acidification based on H⁺ ions in the electrolyte solution holding part 22 are suppressed. Therefore, an inexpensive carbon electrode without any possibility of the elution of metal ions can be used preferably in place of the active electrode such as a silver/silver chloride couple electrode.

The electrolyte solution holding parts 12, 22, and 24 in the iontophoresis device X1 in FIG. 1 hold an electrolyte solution for keeping the conductivity. Phosphate buffered saline, physiological saline, etc. can be used as the electrolyte solution typically.

Furthermore, in order to more effectively prevent the generation of gas caused by the electrolytic reaction of water and the increase in a conductive resistance caused by the generation of gas, or the change in pH caused by the electrolytic reaction of water, a compound that is more easily to be oxidized or reduced than the electrolytic reaction (oxidation at the positive pole and the reduction at the negative pole) of water can be added to the electrolyte solution holding parts 12 and 22. In terms of the biological safety and economic efficiency (low cost and easy availability), for example, an inorganic compound such as ferrous sulfate or ferric sulfate, a medical agent such as ascorbic acid (vitamin C) or sodium ascorbate, an acidic compound that is present on the surface of a skin such as lactic acid, and an organic acid such as oxalic acid, malic acid, succinic acid, or fumaric acid and/or a salt thereof can be used preferably. Alternatively, a combination of those substances (for example, 1:1 mixed aqueous solution containing 1 mol (M) of lactic acid and 1 mol (M) of sodium fumarate) can also be used.

Regarding the electrolyte solution holding part 12, in order to avoid the change in composition owing to the mixture with a drug solution held in a drug holding part 14 (described later), the drug solution having the same composition as that of the drug solution held in the drug holding part 14 can also be used.

Regarding the electrolyte solution holding parts 22 and 24, in order to avoid the change in composition owing to the mixture of an electrolyte solution in the electrolyte solution holding parts 22 and 24, the electrolyte solution with the same composition can be used.

The electrolyte solution holding parts 12, 22, and 24 may hold the above-mentioned electrolyte solution in a liquid state. However, the electrolyte solution holding parts 12, 22, and 24 may be configured by impregnating a water-absorbing thin film carrier made of a polymer material or the like with the above-mentioned electrolyte solution, thereby enhancing the ease of handling thereof. The same thin film carrier as that can be used in the drug holding part 14 can also be used as the thin film carrier used herein. Therefore, the detail thereof will be described in the following description regarding the drug holding part 14.

The drug holding part 14 in the iontophoresis device X1 according to this embodiment holds at least an aqueous solution of a drug (for example, ascorbic acid) whose active ingredient is dissociated to minus ions by the dissolution, as a drug solution.

Here, the drug holding part 14 may hold a drug solution in a liquid state. However, it is also possible to impregnate such a water-absorbing thin film carrier as described below with a drug solution so as to enhance the ease of handling thereof.

Examples of a material that can be used for the water-absorbing thin film carrier in this case include a hydrogel body of acrylic-based resin (acrylhydrogel film), segmented polyurethane-based gel film, and an ion conductive porous sheet for forming a gel solid electrolyte. By impregnating the above aqueous solution at an impregnation ratio of 20 to 60%, a high transport number (high drug delivery property), e.g., 70 to 80% can be obtained.

The impregnation ratio in the present specification is represented by % by weight (i.e., 100 x (W-D)/D[%] where D is a weight in a dry state and W is a weight after impregnation). The impregnation ratio should be measured immediately after the impregnation with an aqueous solution to eliminate an influence with time.

Furthermore, the transport number in the present specification refers to the ratio of a current that contributes to the transfer of drug ions among all the currents flowing through the working electrode structure. The transport number should be measured in such a manner that another constituent member is assembled with a thin film impregnated with a drug solution interposed between the ion-exchange membranes 13 and 15 so as to minimize the change with time.

Herein, the above-mentioned acrylhydrogel film (for example, available from Sun Contact Lens Co., Ltd.) is a gel body having a three-dimensional network structure (cross-linking structure). When an electrolyte solution that is a dispersion medium is added to the acrylhydrogel film, the acrylhydrogel film becomes a polymer adsorbent having ion conductivity. Furthermore, the relationship between the impregnation ratio of the acrylhydrogel film and the transport number can be adjusted depending upon the size of the three-dimensional network structure and the kind and ratio of a monomer constituting a resin. The acrylhydrogel film with an impregnation ratio of 30 to 40% and a transport number of 70 to 80% can be prepared from 2-hydroxyethylmethacrylate and ethyleneglycol dimethacrylate (monomer ratio 98 to 99.5 : 0.5 to 2), and it is confirmed that the impregnation ratio and transport number are almost the same in a range of an ordinary thickness of 0.1 to 1 mm.

Furthermore, the segmented polyurethane gel film has, as segments, polyethylene glycol (PEG) and polypropylene glycol (PPG), and can be adjusted based on a monomer and diisocyanate constituting these segments. The segmented polyurethane gel film has a three-dimensional structure cross-linked by a urethane bond, and the impregnation ratio, transport number, and adhesion strength of the gel film can be easily adjusted by controlling the size of a network, and the kind and ratio of a monomer in the same way as in the acrylhydrogel film. When water that is a dispersion medium and an electrolyte (alkaline metal salt, etc.) are added to the segmented polyurethane gel film (porous gel film), oxygen in an ether connecting part of polyether forming a segment and an alkaline metal salt form a complex, and ions of the metal salt move to oxygen in a subsequent blank ether connecting part when a current flows, whereby the conductivity is expressed.

As the ion conductive porous sheet for forming a gel solid electrolyte, for example, there is the one disclosed in JP 11-273452 A. This porous sheet is based on an acrylonitrile copolymer, and a porous polymer with a porosity of 20 to 80%. More specifically, this porous sheet is based on an acrylonitrile-based copolymer with a porosity of 20 to 80% containing 50 mol% or more (preferably 70 to 98 mol%) of acrylonitrile. The acrylonitrile-based gel solid electrolytic sheet (solid-state battery) is prepared by impregnating an acrylonitrile-based copolymer sheet soluble in a non-aqueous solvent and having a porosity of 20 to 80%, with a non-aqueous solvent containing an electrolyte, followed by gelling, and a gel body includes a gel to a hard film.

In terms of the ion conductivity, safety, and the like, the acrylonitrile-based copolymer sheet soluble in a non-aqueous solvent is preferably composed of an acrylonitrile/C 1 to C4 alkyl (meth)acrylate copolymer, an acrylonitrile/vinylacetate copolymer, an acrylonitrile/styrene copolymer, an acrylonitrile/vinylidene chloride copolymer, or the like. The copolymer sheet is made porous by an ordinary method such as a wet (dry) paper making method, a needlepunching method that is a kind of a non-woven fabric producing method, a water-jet method, drawing perforation of a melt-extruded sheet, or perforation by solvent extraction. In the present invention, among the above-mentioned ion conductive porous sheets of an acrylonitrile-based copolymer used in a solid-state battery, a gel body (a gel to a hard film) holding the above-mentioned aqueous solution in a three-dimensional network of a polymer chain and in which the above-mentioned impregnation ratio and transport number are achieved is useful as a thin film carrier used in the drug holding part 14 or the electrolyte solution holding parts 12, 22, and 24 of the present invention.

In the present invention, regarding the conditions for impregnating the above-mentioned thin film carrier with a drug solution or an electrolyte solution, the optimum conditions may be determined in terms of the impregnation amount, impregnation speed, and the like. For example, an impregnation condition of 30 minutes at 40°C may be selected.

In the iontophoresis device X1 according to the present embodiment, an ion-exchange membrane in which a part or an entirety of the pores of the porous film is filled with ion-exchange resin having a cation exchange function can be used as the cation exchange membranes 13 and 25, and an ion-exchange membrane filled with ion-exchange resin having an anion exchange function can be used as the anion exchange membranes 15 and 23. For example, NEOSEPTA, CM-1, CM-2, CMX, CMS, CMB, CLE04-2, etc. produced by Tokuyama Co., Ltd. can be used as the cation exchange membranes 13 and 15. NEOSEPTA, AM-1, AM-3, AMX, AHA, ACH, ACS, ALE04-2, AIP-21, etc. produced by Tokuyama Co., Ltd. can be used as the ion-exchange membranes 15 and 23.

Herein, a fluorine type resin with an ion-exchange group introduced to a perfluorocarbon skeleton or a hydrocarbon type resin containing a resin that is not fluorinated as a skeleton can be used as the above-mentioned ion-exchange resin. In view of the convenience of a production process, a hydrocarbon type ion-exchange resin is preferable. Furthermore, although the filling ratio of the ion-exchange resin is also related to the porosity of the porous film, the filling ratio is generally 5 to 95% by mass, in particular, 10 to 90% by mass, and preferably 20 to 60% by mass.

There is no particular limit to an ion-exchange group of the above-mentioned ion-exchange resin, as long as it is a functional group generating a group having negative or positive charge in an aqueous solution. As specific examples of the functional group to be such an ion-exchange group, those of a cation exchange group include a sulfonic acid group, a carboxylic acid group, and a phosphonic acid group. Those acid groups may be present in the form of a free acid or a salt. Examples of a counter cation in the case of a salt include alkaline metal cations such as sodium ions and potassium ions, and ammonium ions. In those cation exchange groups, generally, a sulfonic acid group that is a strong acidic group is particularly preferable. Examples of the anion exchange group include primary to tertiary amino groups, a quaternary ammonium group, a pyridyl group, an imidazole group, a quaternary pyridinium group, and a quaternary imidazolium group. Examples of a counter anion in those anion exchange groups include halogen ions such as chlorine ions and hydroxy ions. In those anion exchange groups, generally, a quaternary ammonium group and a quaternary pyridinium group that are strong basic groups are used preferably.

As the above-mentioned porous film, it is possible to use a film or a sheet having a number of pores communicating a front side and a back side thereof, such as a single film formed by extrusion, calendaring, or the like in which the pores in the form of continuous air holes capable of carrying the ion-exchange resin are formed, and a film formed of woven fabric or non-woven fabric of a fibrous material in which the ion-exchange resin is carried in the pores formed by a gap between fibers, without any particular limit. In order to satisfy both high strength and flexibility, it is preferable that the porous film is made of thermoplastic resin.

Examples of the thermoplastic resins constituting the porous film include, without limitation: polyolefin resins such as homopolymers or copolymers of α-olefins such as ethylene, propylene, 1-butene, 1-pentene, 1-hexene, 3-methyl-1-butene, 4-methyl-1-pentene, and 5-methyl-1-heptene; vinyl chloride resins such as polyvinyl chloride, vinyl chloride-vinyl acetate copolymers, vinyl chloride-vinylidene chloride copolymers, and vinyl chloride-olefin copolymers; fluorine resins such as polytetrafluoroethylene, polychlorotrifluoroethylene, polyvinylidene fluoride, tetrafluoroethylene-hexafluoropropylene copolymers, tetrafluoroethylene-perfluoroalkyl vinylether copolymers, and tetrafluoroethylene-ethylene copolymers; polyamide resins such as nylon 6 and nylon 66; and those which are made from polyamide resins. Polyolefin resins are preferably used as they are superior in mechanical strength, flexibility, chemical stability, and chemical resistance, and have good compatibility with ion-exchange resins. As the polyolefin resins, polyethylene and polypropylene are particularly preferable and polyethylene is most preferable.

There is no particular limit to the property of the above-mentioned porous film made of the thermoplastic resin. However, the average pore diameter of the pores may be preferably 0.005 to 5.0 µm, more preferably 0.01 to 2.0 µm, and most preferably 0.02 to 0.2 µm since the porous film having such an average pore diameter is likely to be a thin ion-exchange membrane having excellent strength and a low electric resistance. The average pore diameter in the present specification refers to an average flow pore diameter measured in accordance with a bubble point method (JIS K3832-1990). Similarly, the porosity of the porous film may be preferably 20 to 95%, more preferably 30 to 90%, and most preferably 30 to 60%. Furthermore, the thickness of the porous film may be preferably 5 to 140 µm, more preferably 10 to 120 µm, and most preferably 15 to 55 µm. Usually, an anion exchange membrane or a cation exchange membrane using such a porous film has a thickness of the porous film with +0 to 20 µm.

Regarding the porous film used for each of the anion exchange membrane 15 and the cation exchange membrane 25, before or after the filling of the ion-exchange resin or the introduction of the ion-exchange group, a water-soluble polymer compound is chemically bonded to the front surface (the surface opposing to the skin 4), whereby a first layer of the water-soluble polymer compound is formed.

Examples of the water-soluble polymer compound include polymer compounds having a molecular weight of about 50 to 1,000 dissolved easily in water, such as polyvinyl alcohol (PVA), carboxymethylcellulose, polyacrylic acid, poly-N-isopropylacrylamide, polyacrylamide, poly-N-methylacrylamide, polyvinylpyrrolidone, polymethacrylic acid, polyethylene glycol, polyethyleneimine, poly-N-dimethylaminoethyl methacrylate, and polypeptide. A water-soluble polymer compound can be chemically bonded to the front surface of the porous film by irradiating the porous film immersed in an aqueous solution in which the water-soluble polymer compound is dissolved, with a UV-ray, an electron beam, a γ-ray, or plasma.

Each of the above-mentioned water-soluble polymer compounds has a straight-chain structure, and by appropriately controlling the reaction condition in the above-mentioned treatment, such as concentration or temperature of the water-soluble polymer compound or the irradiation amount of a UV-ray, an electron beam, a γ-ray, or plasma, the water-soluble polymer compound can be bonded to the front surface of the porous film in a brush shape. Depending upon the reaction condition, the first layers can be formed in such a way that a single molecule of the water-soluble polymer compound is bonded to the front surface of the porous film in a brush shape, or in such a way that a plurality of (several to tens of) water-soluble polymer compounds connected in a chain-length direction are bonded to the front surface of the porous film in a brush shape.

As the anion exchange membrane 15 and the cation exchange membrane 25, a porous film can also be used in which a second layer made of a water-soluble polymer compound is laminated on the first layer of the water-soluble polymer compound formed as described above.

As the water-soluble polymer compound used for the second layer, it is possible to use polymer compounds having a molecular weight of, for example, about 1,000 to 1,000,000 dissolved easily in water, such as polyvinyl alcohol (PVA), carboxymethylcellulose, polyacrylic acid, poly-N-isopropylacrylamide, polyacrylamide, poly-N-methylacrylamide, polyvinylpyrrolidone, polymethacrylic acid, polyethylene glycol, polyethyleneimine, poly-N-dimethylaminoethyl methacrylate, and polypeptide. The second layer can be formed by coating and drying an aqueous solution of these water-soluble polymer compounds diluted to an appropriate concentration by a usual method such as spin coating or bar coating.

It is preferable that the thickness of the second layer made of a water-soluble polymer compound is in a range of 1 to 200 µm.

FIG. 2 is a conceptual explanatory view showing a state where an ion-exchange membrane 15 using a porous film 15a with a first layer M1 of a water-soluble polymer compound bonded to the front surface thereof in a brush shape is in contact with a skin 4 having concave portions 4a and convex portions 4b.

As shown, in the case of using the ion-exchange membrane 15 for the working electrode structure 1, owing to the presence of the first layer M1 of the water-soluble polymer compound bonded to the front surface of the porous film 15a in a brush shape, the contact area with respect to the skin 4 increases. Furthermore, the water of a drug holding part 14 bleeding out from openings 15b of the porous film 15a is held in the first layer M1, whereby the adhesion and conductivity between the skin 4 and the ion-exchange membrane 15 are expected to be enhanced.

Simultaneously, portions such as the convex portions 4b of the skin close to the openings 15b of the porous film 15a hold the original function of an iontophoresis device for administering drug ions via an ion-exchange membrane, i.e., the function of suppressing the release of biological counter ions from the skin 4 by the ion-exchange resin 15c being filled in the pores of the porous film 15a, while allowing drug ions to move from the ion-exchange resin 15c to the skin 4.

In the concave portions 4a of the skin 4, a layer of the water bleeding out from the openings 15b is formed, and consequently, the adhesion and the conductivity between the skin 4 and the ion-exchange membrane 15 are expected to be enhanced. On the other hand, a part of charge supplied to the working electrode structure is considered to be consumed by the release of biological counter ions from the skin 4 to the layer of the water. However, the volume of such water layer is very small, so that the charge amount consumed by this release is considered to be negligible.

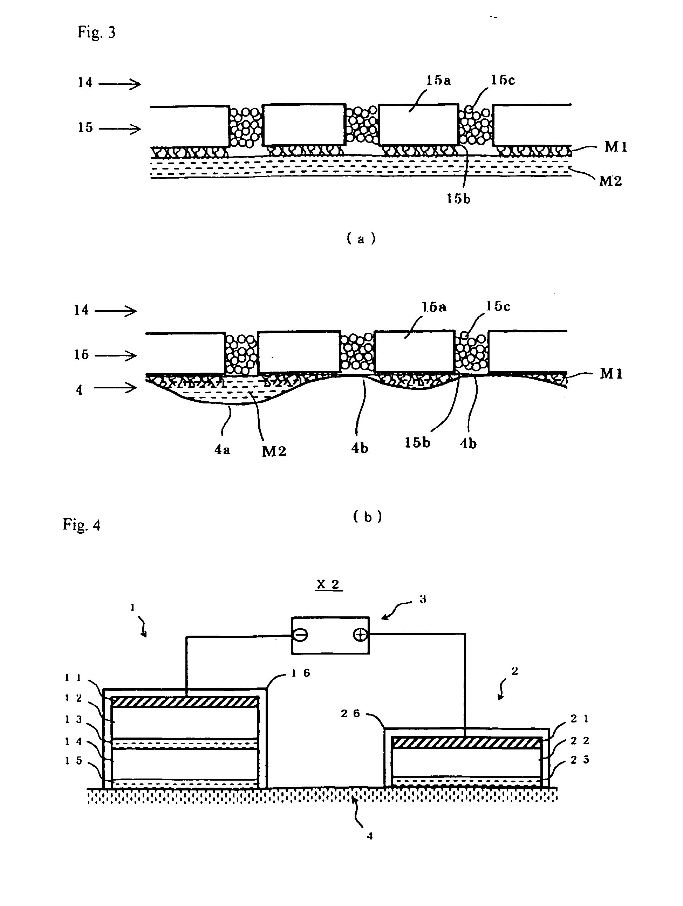

FIG. 3A is a conceptual explanatory view showing an ion-exchange membrane 15 using a porous film 15a with a second layer M2 made of a water-soluble polymer compound further formed on a first layer M1 of the water-soluble polymer compound similar to that of FIG. 2. FIG. 3B is a conceptual explanatory view showing a state where the ion-exchange membrane 15 is in contact with the skin 4 having concave portions 4a and convex portions 4b.

As shown in FIG. 3A, the second layer M2 is formed to some thickness on the first layer M1. The second layer M2 is capable of flowing easily owing to the water bleeding out from the openings 15b. When the ion-exchange membrane 15 is brought into contact with the skin 4, as shown in FIG. 3B, the water-soluble polymer compound between the convex portions 4b of the skin and the ion-exchange membrane 15 moves to the space between the concave portions 4a and the ion-exchange membrane 15.

Therefore, the adhesion and the conductivity between the ion-exchange membrane 15 and the skin 4 become more satisfactory. On the other hand, the second layer M2 is not present between the convex portions 4b of the skin and the ion-exchange membrane 15, or is present as a very thin film. Therefore, the convex portions 4b of the skin close to the openings 15b of the porous film 15a hold the original function of an iontophoresis device for administering drug ions via an ion-exchange membrane, i.e., the function of suppressing the release of biological counter ions from the skin 4 by the ion-exchange resin 15c, while allowing drug ions to move from the ion-exchange resin 15c to the skin 4.

For the ion-exchange membrane 25 of the nonworking electrode structure 2, it is also possible to use a porous film having a first layer of a water-soluble polymer compound bonded to the front surface in a brush shape in the same way as the above, or a second layer of a water-soluble polymer compound further laminated on the first layer in the same way as the above, whereby the adhesion and conductivity between the ion-exchange membrane 25 and the skin 4 can be enhanced.

As the power source 3 in the iontophoresis device of the present invention, a battery, a voltage stabilizer, a current stabilizer (galvano device), a voltage/current stabilizer, or the like can be used. It is preferable to use a current stabilizer that is operated under safe voltage conditions in which an arbitrary current can be adjusted in a range of 0.01 to 1.0 mA, preferably 0.01 to 0.5 mA, specifically, at 50V or less, preferably, 30 V or less.

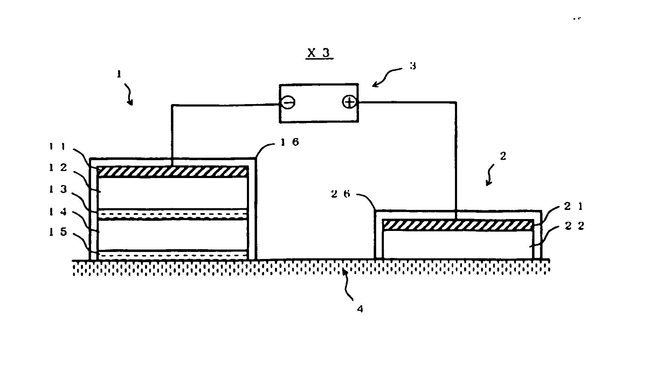

FIGS. 4 and 5 are explanatory views showing configurations of iontophoresis devices X2 and X3 according to other embodiments.

The iontophoresis device X2 in FIG. 4 has the same configuration as that of the iontophoresis device X1 shown in FIG. 1 except that the nonworking electrode structure 2 does not have the ion-exchange membrane 23 and the electrolyte solution holding part 24. The iontophoresis device X3 in FIG. 5 has the same configuration as that of the iontophoresis device X1 shown in FIG. 1 except that the nonworking electrode structure 2 does not have the ion-exchange membranes 23 and 25, and the electrolyte solution holding part 24. Although the iontophoresis devices X2 and X3 are not comparable to the iontophoresis device X1 in terms of the performance of suppressing the change in pH on the contact surface of the nonworking electrode structure 2 with respect to the skin 4, they exhibit performance equal to that of the iontophoresis device X1 in the other points. In particular, owing to the anion exchange membrane 15 and/or the cation exchange membrane 25 having the first layers M1 of a water-soluble polymer compound or further the second layer M2 on the surface front thereof, the iontophoresis devices X2 and X3 exhibit the functional effect peculiar to the present invention of stably administering drug ions at a high efficiency, compared with the conventional iontophoresis devices.

The present invention has been described with reference to the embodiment. The present invention is not limited thereto, and various alterations can be made within the scope of the claims.

For example, in the above embodiment, the case has been described in which the working electrode structure 1 has the electrolyte solution holding part 12 and the ion-exchange membrane 13 in addition to the electrode 11, the drug holding part 14, and the ion-exchange membrane 15. However, the electrolyte holding part 12 and the ion-exchange membrane 13 can be omitted. In this case, the function of suppressing the decomposition of a drug in the vicinity of the electrode 11, the function of suppressing the movement of H⁺ ions and OH⁻ ions to the skin interface or the change in pH at the skin interface caused by the movement of ions, and the like are not comparable to those in the above-mentioned embodiment. However, the enhancement of the administration efficiency of drug ions to a living body, which is a basic functional effect of the present invention, is similarly achieved. Such an iontophoresis device is also included in the scope of the present invention.

Similarly, in the nonworking electrode structure 2, the following may be possible. The electrolyte solution holding parts 22 and 24, and the ion-exchange membranes 23 and 25 are omitted, and for example, the electrode 21 of the nonworking electrode structure 2 is brought into direct contact with the skin coated with an electrolyte solution, whereby a drug is administered. Furthermore, the nonworking electrode structure 2 is not provided in an iontophoresis device, and for example, the working electrode structure 1 is brought into contact with the skin of a living body, while a voltage is applied to the working electrode structure 1 under the condition that a part of the living body is kept in contact with a member to be the earth, whereby a drug is administered. In this case, the performance of suppressing the change in pH at the interface between the electrode 21 or the member to be the earth, and the skin is not comparable to that in the above-mentioned embodiment. However, the enhancement of the administration efficiency of drug ions to a living body, which is a basic functional effect of the present invention, is similarly achieved. Such an iontophoresis device is also included in the scope of the present invention.

Furthermore, in the above embodiment, the case has been described where the working electrode structure 1, the nonworking electrode structure 2, and the power source 3 are configured separately. It is also possible that those elements are incorporated in a single casing or an entire device incorporating them is formed in a sheet shape or a patch shape, whereby the handling thereof is enhanced, and such an iontophoresis device is also included in the scope of the present invention.

### BREIF DESCRIPTION OF THE DRAWINGS

[Fig. 1] FIG. 1 is a schematic cross-sectional view showing a basic configuration of an iontophoresis device according to one embodiment of the present invention;
[Fig. 2] FIG. 2 is a conceptual explanatory view schematically showing a situation in which an ion-exchange membrane used in the iontophoresis device of the present invention is in contact with the skin;
[Fig. 3] (a) is a conceptual explanatory view schematically showing an ion-exchange membrane used in the iontophoresis device of the present invention, and (b) is a conceptual explanatory view schematically showing a situation in which the ion-exchange membrane is in contact with the skin;
[Fig. 4] FIG. 4 is an explanatory view showing a configuration of an iontophoresis device according to another embodiment of the present invention; and
[Fig. 5] FIG. 5 is an explanatory view showing a configuration of an iontophoresis device according to another embodiment of the present invention.

## Claims

1. An iontophoresis device (X1; X2; X3) comprising a working electrode structure (1) comprising:
a first ion-exchange membrane (15) including a first porous film (15a) with a number of pores (15b) communicating a front surface and a back surface of the first porous film (15a) and an ion-exchange resin (15c) filled in the pores (15b) of the first porous film (15a), said first ion-exchange membrane (15) selecting ions of a first conductivity; and
a drug holding part (14) placed on a back surface side of the first ion-exchange membrane (15) and holding a drug solution containing drug ions charged in the first conductivity, the drug ions being administered via the first ion-exchange membrane (15),
**characterized in that** a first layer (M1) of a water-soluble polymer compound chemically bonded to a material constituting the first porous film (15a) is formed on the front surface of the first porous film (15a).

2. An iontophoresis device (X1; X2) according to claim 1, further comprising a nonworking electrode structure (2) operating as a counterpart of the working electrode structure (1),
the nonworking electrode (2) structure having:
a second ion-exchange membrane (25) including a second porous film with a number of pores communicating a front surface and a back surface of the second porous film and an ion-exchange resin filled in the pores of the second porous film, said second ion-exchange membrane (25) selecting ions of a second conductivity opposite to the first conductivity; and
a first electrolyte solution holding part (24) for holding an electrolyte solution, placed on a back surface side of the second ion-exchange membrane (25),
**characterized in that** a first layer (M1) of a water-soluble polymer compound chemically bonded to a material constituting the second porous film is formed on the front surface of the second porous film.

3. An iontophoresis device (X1; X2; X3) according to claim 1 or 2, **characterized in that**:
the working electrode structure (1) further comprises:
a first electrode (11) supplied with a voltage of the first conductivity;
a second electrolyte solution holding part (12) for holding an electrolyte solution in electrically contact with the first electrode (11); and
a third ion-exchange membrane (13) placed on a front surface side of the second electrolyte holding part (12) and selecting ions of the second conductivity; and that
the drug holding part (14) is placed on a front surface side of the third ion-exchange membrane (13), and
the first ion-exchange membrane (15) is placed on a front surface side of the drug holding part (14).

4. An iontophoresis device (X1; X2; X3) according to any one of claims 1 to 3, **characterized in that** a water-soluble polymer compound constituting the first layer (M1) has a straight-chain structure, and is bonded to the front surface of one of the first porous film (15a) and the second porous film in a brush shape.

5. An iontophoresis device (X1; X2; X3) according to any one of claims 1 to 4, **characterized in that** the first layer (M1) is formed by irradiating one of the first porous film (15a) and the second porous film immersed in an aqueous solution in which a water-soluble polymer compound is dissolved, with a UV-ray, an electron beam, a γ-ray, or plasma.

6. An iontophoresis device (X1; X2; X3) according to any one of claims 1 to 5, **characterized by** further comprising a second layer (M2) made of a water-soluble polymer compound laminated on the first layer (M1) of the water-soluble polymer compound on the front surface of one of the first porous film (15a) and the second porous film.

7. An iontophoresis device (X1; X2; X3) according to any one of claims 1 to 6, **characterized in that** the water-soluble polymer compound forming the first layer (M1) and/or the second layer (M2) is one selected from the group consisting of: polyvinyl alcohol; poly-N-isopropylacrylamide; polyacrylic acid; poly-N-methylacrylamide; polyacrylamide; poly-N-methylacrylamide; polyvinylpyrrolidone; polymethacrylic acid; polyethylene glycol; polyethyleneimine; poly-N-dimethylaminoethyl methacrylate; and polypeptide; or a mixture thereof.

## Patentansprüche

1. Eine Iontophoresevorrichtung (X1; X2; X3), die eine Arbeitselektrodenstruktur (1), aufweist, welche folgendes aufweist:
Eine erste Ionenaustauschmembran (15) beinhaltend einen ersten porösen Film (15a) mit mehreren Löchern (15b), die im Austausch stehen mit einer Vorderseite und einer Rückseite des ersten porösen Films (15a), und ein Ionenaustauschharz (15c), das in die Löcher (15b) des ersten porösen Films (15a) gefüllt ist, wobei die erste Ionenaustauschmembran (15) Ionen mit einer ersten Leitfähigkeit auswählt; und
ein Arzneiaufnahmeteil (14), das an einer Rückseite der ersten Ionenaustauschmembran (15) angeordnet ist, und eine Arzneilösung, beinhaltend Arzneiionen, die eine erste Leitfähigkeit aufweisen, wobei die Arzneiionen durch die erste Ionenaustauschmembran (15) dosiert werden,
**dadurch gekennzeichnet, dass** eine erste Schicht (M1) einer wasserlöslichen Polymerverbindung, die chemisch mit einem Material welches den ersten porösen Film (15a) bildet, verbunden ist, an der vorderen Fläche des ersten porösen Films (15a) gebildet wird.

2. Iontophoresevorrichtung (X1; X2) nach Anspruch 1, weiterhin aufweisend eine nicht-Arbeitselektrodenstruktur (2), die als Gegenstück zu der Arbeitselektrodenstruktur (1) arbeitet, wobei die nicht-Arbeitselektrodenstruktur (2) folgendes aufweist:
eine zweite Ionenaustauschmembran (25) beinhaltend einen zweiten porösen Film mit mehreren Löchern, die im Austausch stehen mit einer Vorderseite und einer Rückseite des zweiten porösen Films und ein Ionenaustauschharz, das in die Löcher des zweiten porösen Films gefüllt ist, wobei die zweite Ionenaustauschmembran (25) Ionen mit einer zweiten Leitfähigkeit anders als die erste Leitfähigkeit auswählt; und
eine erste Elektrolytlösungsaufnahme (24), die eine Elektrolytlösung aufweist, angeordnet an der Rückseite der zweiten Ionenaustauschmembran (25),
**dadurch gekennzeichnet, dass** eine erste Schicht (M1) einer wasserlöslichen Polymerverbindung, die chemisch mit einem Material, welches den zweiten porösen Film bildet, verbunden ist, an der vorderen Fläche des zweiten porösen Films gebildet wird.

3. Iontophoresevorrichtung (X1; X2; X3) nach einem der Ansprüche 1 oder 2, **dadurch gekennzeichnet, dass**:
die Arbeitselektrodenstruktur (1) weiterhin folgendes aufweist:
eine erste Elektrode (11), die mit einer Spannung der ersten Leitfähigkeit gespeist wird;
ein zweites Elektrolytlösungsaufnahmeteil (12), um eine Elektrolytlösung in elektrischen Kontakt mit der ersten Elektrode (11) zu halten; und
eine dritte Ionenaustauschmembran (13), angeordnet an der vorderen Flächenseite des zweiten Elektrolytlösungsaufnahmeteils (12) und die Ionen der zweiten Leitfähigkeit auswählend; und dass
das Arzneiaufnahmeteil (14) an der vorderen Flächenseite der dritten Ionenaustauschmembran (13) angeordnet ist, und
die erste Ionenaustauschmembran (15) an der vorderen Seitenfläche des Arzneiaufnahmeteils (14) angeordnet ist.

4. Iontophoresevorrichtung (X1; X2; X3) nach einem der Ansprüche 1 bis 3, **dadurch gekennzeichnet, dass** eine wasserlöslichen Polymerverbindung, die die erste Schicht (M1) bildet, eine geradlinige Kettenstruktur hat, und an der vorderen Fläche des ersten porösen Films (15a) und des zweiten porösen Films in Bürstenform gebunden ist.

5. Iontophoresevorrichtung (X1; X2; X3) nach einem der Ansprüche 1 bis 4, **dadurch gekennzeichnet, dass** die erste Schicht (M1) gebildet wird, indem einer des ersten porösen Films (15a) und des zweiten porösen Films in eine wässrige Lösung getaucht wird, in dem eine wasserlösliche Polymerverbindung gelöst ist, und mit UV-Strahlung, einem Elektronenstrahl, einem Gammastrahl oder mit Plasma bestrahlt wird.

6. Iontophoresevorrichtung (X1; X2; X3) nach einem der Ansprüche 1 bis 5, **dadurch gekenzeichnet, dass** sie ferner eine zweite Schicht (M2) einer wasserlöslichen Polymerverbindung auf der ersten Schicht (M1) der wasserlöslichen Polymerverbindung auf der vorderen Seite von einer des ersten porösen Films (15a) und des zweiten porösen Films aufweist.

7. Iontophoresevorrichtung (X1; X2) nach einem der Ansprüche 1 bis 6, **dadurch gekennzeichnet, dass** die wasserlösliche Polymerverbindung, die die erste Schicht (M1) und/ oder die zweite Schicht (M2) bildet, eine aus der Gruppe ist, die besteht aus: Polyvinyl Alkohol; Poly-N-Isopropylacrylamid; Polyacrylsäure; Poly-M-Methylacrylamid; Polyacrylamid; Poly-N-Methylacrylamid; Polyvinylpyrrolidon; Polymethacrylsäure; Polyethylenglycol; Polyethyleneimin; Poly-N-Dimethylaminoehtyl Methacrylat; und Polypeptid oder einer Mischung davon.

## Revendications

1. Un dispositif d'iontophorèse (X1 ; X2 ; X3) doté d'une structure d'électrode active (1) composée :
d'une première membrane d'échange ionique (15) comportant un premier film poreux (15 a) doté d'un certain nombre de pores (15 b) qui permettent à une surface avant et une surface arrière du premier film poreux (15 a) de communiquer, et une résine d'échange ionique (15 c) placée dans les pores (15 b) du premier film poreux (15 a), ladite membrane d'échange ionique (15) sélectionnant les ions d'un premier type de conductivité ; et
d'une unité contenant un médicament (14) disposée sur un côté de la surface arrière de la première membrane d'échange ionique (15) et contenant une solution médicamenteuse contenant des ions médicamenteux d'un premier type de conductivité, les ions médicamenteux étant administrés via la première membrane d'échange ionique (15), **caractérisé en ce qu'**une première strate (M1) d'un composé polymère soluble à l'eau lié chimiquement à un matériau constituant le premier film poreux (15 a) est formée sur la surface avant du premier film poreux (15 a).

2. Un dispositif d'iontophorèse (X1; X2) selon la Revendication 1, comprenant en outre une structure d'électrode inactive (2) fonctionnant comme une contrepartie de la structure d'électrode active (1),
la structure d'électrode inactive (2) disposant de :
une seconde membrane d'échange ionique (25) comportant un second film poreux doté d'un certain nombre de pores qui permettent à une surface avant et une surface arrière du second film poreux de communiquer, et une résine d'échange ionique placée dans les pores du second film poreux, ladite seconde membrane d'échange ionique (25) sélectionnant les ions d'un second type de conductivité opposé au premier type de conductivité ; et
un premier conteneur de solution électrolytique (24) destiné à contenir une solution électrolytique, placé sur un côté de la surface arrière de la seconde membrane d'échange ionique (25),
**caractérisé en ce qu'**une première strate (M1) d'un composé polymère soluble à l'eau lié chimiquement à un matériau constituant le second film poreux est formée sur la surface avant du second film poreux.

3. Un dispositif d'iontophorèse (X1 ; X2 ; X3) selon la Revendication 1 ou 2, **caractérisé en ce que** :
la structure d'électrode active (1) comprend en outre :
une première électrode (11) présentant un voltage du premier type de conductivité :
un second conteneur de solution électrolytique (12) destiné à maintenir une solution électrolytique en contact électrique avec la première électrode (11) ; et
une troisième membrane d'échange ionique (13) placée sur un côté de la surface avant du second conteneur de solution électrolytique (12) et qui sélectionne les ions du second type de conductivité ; et **en ce que**
l'unité contenant un médicament (14) est disposée sur un côté de la surface avant de la troisième membrane d'échange ionique (13), et
la première membrane d'échange ionique (15) est placée sur un côté de la surface avant de l'unité contenant un médicament (14).

4. Un dispositif d'iontophorèse (X1 ; X2 ; X3) selon l'une quelconque des Revendications 1 à 3, **caractérisé en ce qu'**un composé polymère soluble à l'eau constituant la première strate (M1) présente une structure à chaîne droite et est lié à la surface avant d'un premier film poreux (15 a) et au second film poreux selon une forme rappelant celle d'une brosse.

5. Un dispositif d'iontophorèse (X1 ; X2 ; X3) selon l'une quelconque des Revendications 1 à 4, **caractérisé en ce que** la première strate (M1) est formée de sorte à irradier de rayons UV, d'un faisceau d'électrons, d'un rayon γ ou plasma un premier film poreux (15 a) et le second film poreux immergé dans une solution aqueuse dans laquelle un composé polymère soluble dans l'eau est dissout.

6. Un dispositif d'iontophorèse (X1 ; X2 ; X3) selon l'une quelconque des Revendications 1 à 5, **caractérisé en ce qu'**il contient en outre une deuxième strate (M2) faite d'un composé polymère soluble dans l'eau laminé sur la première strate (M1) du composé polymère soluble à l'eau sur la surface avant d'un premier film poreux (15 a ) et du second film poreux.

7. Un dispositif d'iontophorèse (X1 ; X2 ; X3) selon l'une quelconque des Revendications 1 à 6, **caractérisé en ce que** le composé polymère soluble formant la première strate M1 et / ou la deuxième strate (M2) est sélectionné parmi le groupe composé de : alcool polyvinyle ; poly N isopropylacrylamide ; acide polyacrylique ; poly N méthylacrylamide ; polyacrylamide ; poly N méthylacrylamide ; polyvinylpyrrolidone ; acide polyméthacrylique ; polyéthylène glycol ; polyéthylèneimine ; méthacrylate de poly N diméthylaminoéthyle ; et polypeptide ; ou présente un mélange de ces composés.
